# EUROPEAN PATENT APPLICATION

(11) **EP 3 381 272 A1**
(43) Date of publication of application: **03.10.2018**
(21) Application number: 17861205.7
(22) Date of filing: 25.04.2017
(51) Int. Cl.: A01H 1/02, B64C 33/00

(54) **MICRO FLYING ROBOT FOR AUTONOMOUS AGRICULTURAL POLLINATION**

(30) Priority: 23.01.2017 CN 201710049123
(71) Applicant: Dongguan University of Technology, Dongguan, Guangdong 523808 (CN); Chen, Yi, Dongguan, Guangdong 523808 (CN); Li, Yun, Dongguan, Guangdong 523808 (CN); Yu, Hongnian, Dongguan, Guangdong 523808 (CN)
(72) Inventor: CHEN, Yi, Dongguan Guangdong 523808 (CN); LI, Yun, Dongguan Guangdong 523808 (CN); YU, Hongnian, Dongguan Guangdong 523808 (CN)
(74) Representative: Bergmeier, Werner
(86) International application number: PCT/CN2017/081879
(87) International publication number: WO 2018/133241

(57) **Abstract**

The present invention discloses a micro air vehicle for autonomous agricultural pollination, which includes a robot body, an intelligent control part, an intelligent sensor part, a battery, a pollination device and a launcher. The robot body includes a body part and a flapping-wing mechanism. The intelligent control part is mounted on a left side of the body part, the intelligent sensor part is mounted on an upper side of the body part, the intelligent control part performs data collection and communication, the battery is mounted on a right side of the body part, the pollination device is connected to a lower side of the body part, and the launcher is mounted in a middle position of the body part. The present invention is more efficient in an automatic process, reducing an operating cost at the same time, being advantageous for propelling agricultural intellectualization, and the present invention can provide a higher efficiency and a better pollination quality and improve an agricultural productivity.

## Description

### TECHNICAL FIELD

The present invention relates to a technical field of robot, and specifically relates to a micro air vehicle (MAV) for autonomous agricultural pollination.

### BACKGROUND

It was in 2006 that US researcher first found a sudden decrease in the number of honeybees. Such situation didn't get better in several years, which still bothers the researchers and bee-keepers until now. According to a statistical data published by United States Department of Agriculture, beehives had decreased by 29% in 2009, decreased by 36% in 2008, and decreased by 32% in 2007. This phenomenon is named "Colony Collapse Disorder" (CCD) by researchers: adult honeybees abandon the honeycombs and keep flying until die. In addition to United States, the phenomenon of CCD also appears in other regions of the world including Europe. Experts consider that reasons which lead to such phenomenon may include parasites, viruses, germs, malnutrition, an abuse of insecticide and the like.

The research shows that about one third of the food for human beings grows depending on pollination by animals and insects, among which honeybees are one of the main pollinators. In particular, a main reason, the global epidemic of CCD, has seriously influenced the food safety of human beings. If the honeybees die out, a short of grain and a famine will appear in the world, which will finally lead to frequent occurrence of violence and riots.

### SUMMARY OF THE INVENTION

An object of the present invention is to provide a micro air vehicle for autonomous agricultural pollination which saves both time and labor and has a good effect, in order to solve the problems stated in the above-described background.

In order to achieve the above-described object, the present invention provides a technical solution below:
A micro air vehicle for autonomous agricultural pollination, comprises a robot body, an intelligent control part, an intelligent sensor part, a battery, a pollination device and a launcher. The robot body comprises a body part and a flapping-wing mechanism. The intelligent control part is mounted on a left side of the body part, the intelligent sensor part is mounted on an upper side of the body part, the intelligent sensor part performs data collection and communication, the battery is mounted on a right side of the body part, the pollination device is connected to a lower side of the body part, and the launcher is mounted in a middle position of the body part.

As a further solution of the present invention: the intelligent control part comprises an intelligent controller and an actuator.

As a further solution of the present invention: the battery supplies energy to the robot body, the intelligent control part, the intelligent sensor part, the battery and the pollination device, and performs energy collection.

As a further solution of the present invention: the pollination device is selected from a group consisting of an active pollination brush, a semi-active pollination brush and a passive pollination brush.

As a further solution of the present invention: a branched structure for capturing is provided on each bristle of the pollination device.

As a further solution of the present invention: the flapping-wing mechanism is a link mechanism or a crankshaft mechanism.

As a furtherer solution of the present invention: the intelligent sensor part comprises a temperature sensor, a speed sensor and an inertial navigation sensor.

Comparing with the prior art, the present invention has beneficial effects:
The present invention is more efficient in an automatic process, reducing an operating cost at the same time, being advantageous for propelling agricultural intellectualization, and the present invention can provide a higher efficiency and a better pollination quality and improve an agricultural productivity.

### BRIEF DESCRIPTION OF THE DRAWINGS

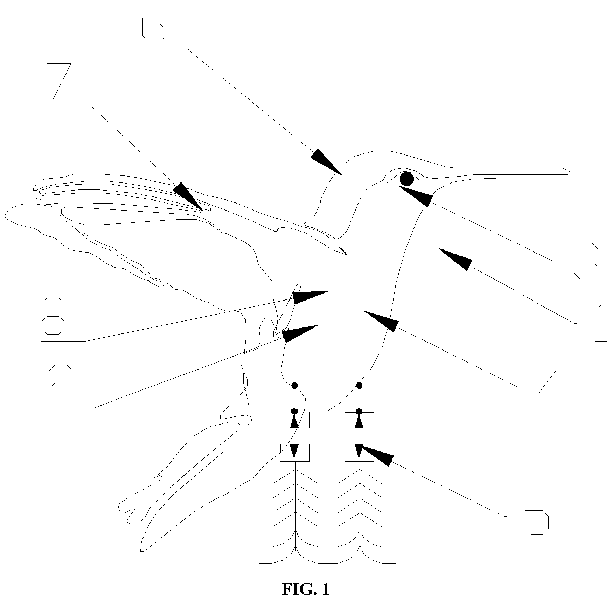

- **FIG. 1**: is a structural schematic diagram of the present invention.

### DETAILED DESCRIPTION OF THE PREFERRED EMBODIMENT

The technical solution of the present application is described in detail below by combining with specific implementation.

As shown in FIG. 1, a micro air vehicle for autonomous agricultural pollination comprised a robot body 1, an intelligent control part 2, an intelligent sensor part 3, a battery 4, a pollination device 5 and a launcher 8. The robot body 1 comprised a body part 6 and a flapping-wing mechanism 7. The intelligent control part 2 was mounted on a left side of the body part 6, the intelligent sensor part 3 was mounted on an upper side of the body part, the intelligent sensor part 3 performed data collection and communication, the battery 4 was mounted on a right side of the body part 6, the pollination device 5 was connected to a lower side of the body part 6, and the launcher 8 was mounted in a middle position of the body part 6.

The intelligent control part 2 comprised an intelligent controller and an actuator; the battery 4 supplied energy to the robot body 1, the intelligent control part 2, the intelligent sensor part 3, the battery 4 and the pollination device 5, and performed energy collection; the pollination device 5 was selected from a group consisting of an active pollination brush, a semi-active pollination brush, and a passive pollination brush; a branched structure for capturing, which can improve an efficiency of pollination, was provided on each bristle of the pollination device 5; the flapping-wing mechanism 7 was a link mechanism or a crankshaft mechanism, and the link mechanism or the crankshaft mechanism was a structural design used for conversing a rotational movement of a motor to a flapping-wing movement 7; and the intelligent sensor part 3 comprised a temperature sensor, a speed sensor and an inertial navigation sensor.

The invention is more efficient in an automatic process, and reduces an operating cost at the same time, no matter the number of wokers increases or decreases. Then agriculture workers, no matter being substituted by other workers or robots, can freely focus on work on which they can exert greater influence and can execute a higher-value work. Besides, requirements for the micro air vehicle for autonomous agricultural pollination create extra work, for example maintenance of a micro air vehicle system for autonomous agricultural pollination. Additionally, an agriculture system will more rely on the micro air vehicle for autonomous agricultural pollination, which is advantageous for propelling agricultural intellectualization.

From an economic point of view, the micro air vehicle for autonomous agricultural pollination provides a higher efficiency and a better pollination quality, and improves an agricultural productivity at the same time. The micro air vehicle system for autonomous agricultural pollination will directly influence the grain production, in order to improve a farming efficiency in the future. For example, in 2009, CCD had influenced 24% of grain crop based on a total yield of crop of UK; and in 2013, CCD had influenced products with value of 4 billion dollars in United States. Products of the micro air vehicle robot for autonomous agricultural pollination will accelerate a development of other industrial products, such as an advanced motor and MEMS system, and will enrich manufacturers or suppliers of advanced materials and industrial engineering.

In order to adjust to the requirements for efficient agricultural automatization in the future, the micro air vehicle for autonomous agricultural pollination, as a substitute for natural pollinators, such as honeybee, insect and the like, can act as an artificial pollinator in an industry of intelligent agriculture, reducing a problem that an ecological correlation, protection and stability of an ecological system, a heritable variation of phytocoenosium of crop, and a diversity, a specialization and an evolution of flowers are seriously influenced due to a decrease in a population quantity of natural pollination medium.

The present invention is more efficient in the automatic process, reducing the operating cost at the same time, being advantageous for propelling agricultural intellectualization, and the present invention can provide a higher efficiency and a better pollination quality and improve the agricultural productivity.

The preferred implementation of the present application is described in detail above, but the present application isn't limited by the above-described implementation. Within the scope of knowledge owned by a person skilled in the art, a plurality of variations can also be made without deviating from the spirit of the present application.

## Claims

1. A micro air vehicle for autonomous agricultural pollination, **characterized in that**, it comprises a robot body (1), an intelligent control part (2), an intelligent sensor part (3), a battery (4), a pollination device (5) and a launcher (8), the robot body (1) comprising a body part (6) and a flapping-wing mechanism (7), the intelligent control part (2) being mounted on a left side of the body part (6), the intelligent sensor part (3) being mounted on an upper side of the body part (6), the intelligent sensor part (3) performing data collection and communication, the battery (4) being mounted on a right side of the body part (6), the pollination device (5) being connected to a lower side of the body part (6), and the launcher (8) being mounted in a middle position of the body part (6).

2. The micro air vehicle for autonomous agricultural pollination according to claim 1, wherein the intelligent control part (2) comprises an intelligent controller and an actuator.

3. The micro air vehicle for autonomous agricultural pollination according to claim 1, wherein the battery (4) supplies energy to the robot body (1), the intelligent control part (2), the intelligent sensor part (3), the battery (4) and the pollination device (5), and performs energy collection.

4. The micro air vehicle for autonomous agricultural pollination according to claim 1, wherein the pollination device (5) is selected from a group consisting of an active pollination brush, a semi-active pollination brush and a passive pollination brush.

5. The micro air vehicle for autonomous agricultural pollination according to claim 1 or 4, wherein a branched structure for capturing is provided on each bristle of the pollination device (5).

6. The micro air vehicle for autonomous agricultural pollination according to claim 1, wherein the flapping-wing mechanism (7) is a link mechanism or a crankshaft mechanism.

7. The micro air vehicle for autonomous agricultural pollination according to claim 1, wherein the intelligent sensor part (3) comprises a temperature sensor, a speed sensor and an inertial navigation sensor.
